# EUROPEAN PATENT SPECIFICATION

(11) **EP 2 142 179 B1**
(45) Date of publication and mention of the grant of the patent: **26.04.2017**
(21) Application number: 07727396.9
(22) Date of filing: 27.03.2007
(51) Int. Cl.: A61K 9/70, A61L 15/44, A61K 33/00

(54) **TOPICAL DERMAL DELIVERY DEVICE FOR NITRIC OXIDE DELIVERY**
TOPISCHE DERMALE ABGABEVORRICHTUNG ZUR ABGABE VON STICKOXID
DISPOSITIF D'ADMINISTRATION DERMIQUE TOPIQUE POUR UNE ADMINISTRATION D'OXYDE NITRIQUE

(43) Date of publication of application: 13.01.2010
(73) Proprietor: BSN medical GmbH, 20253 Hamburg (DE)
(72) Inventor: LINDGREN, Lars, S-252 40 Helsingborg (SE); MÅRLIND, Linda, S-252 34 Helsingborg (SE); THORSEN, Rikard, S-224 74 Lund (SE)
(74) Representative: Jostarndt Patentanwalts-AG
(86) International application number: PCT/EP2007/052923
(87) International publication number: WO 2008/116497

(56) References cited:
- WO-A-02/20026
- WO-A-2006/073520
- WO-A-2006/084909
- WO-A-2006/084910
- WO-A-2006/100154
- WO-A-2006/100155
- WO-A1-98/31315
- US-A- 5 648 101
- HARDWICK J B J ET AL: "A NOVEL METHOD FOR THE DELIVERY OF NITRIC OXIDE THERAPY TO THE SKIN OF HUMAN SUBJECTS USING A SEMI-PERMEABLE MEMBRANE", CLINICAL SCIENCE, BIOCHEMICAL SOCIETY AND THE MEDICAL RESEARCH SOCIETY, LONDON, GB, vol. 100, no. 4, 1 April 2001 (2001-04-01) , pages 395-400, XP001056039, ISSN: 0143-5221, DOI: DOI:10.1042/CS20000187

## Description

### Field of the Invention

This invention pertains in general to the field of topical delivery devices for topical treatment. More particularly, the invention relates to topical dermal delivery devices, such as patches, for topical delivery of nitric oxide (NO) to treatment sites of mammals.

### Background of the Invention

Various compounds and delivery devices for topical treatment of numerous conditions are known.

For instance, US4781924, of Alza Corporation, discloses a diffusional drug delivery system for a therapeutical agent with a delayed onset of drug administration. Moisture activates the system, whereby the drug is converted to a form, which is suitable for absorption through the skin or mucosa. The only forms of activated drugs that are disclosed are free acid, free base and ester. Furthermore, a membrane of the system disclosed, does not provide selective permeation of specific components, such that water and water soluble compounds, as well as rest products of the conversion process, may penetrate through the membrane and into the skin of the user. This may lead to undesired side effects on the user side.

Specific compounds generating nitric oxide (NO) for topical treatment of numerous conditions are known.

WO2006/058318, of The University of Akron, is related to topical nitric oxide delivery systems, and to using the same for mitigating or remediating various disease states. A patch system is disclosed in WO2006/058318, whereby no details are disclosed concerning the design of the patch with regard to a backing or an arrangement towards the skin of the user. The patch comprises a fibrous component, which contains a nitric oxide-releasing agent that is activated by moisture or water. In addition, if such a device is desired to be stored, extreme measures are needed to make sure that NO is not released premature from the patch during storage, e.g. by ensuring a non-moist, non-oxygen and dark storage. In fact many of the materials mentioned in WO2006/058318 usually contain enough moisture themselves to initiate a premature NO release from an NO donor comprised in the patch. Thus, shelf life of such a patch is very limited. Furthermore, both the NO donor disclosed in WO2006/058318, in it self, and the disclosed acid activator could be irritating to the skin, which is disadvantageous for a patch. No measures are disclosed in WO2006/058318 concerning how to avoid skin contact of these components.

WO2003/000088, of Procter & Gamble, discloses a dosing device where a fluid containing an active compound is separated from the rest of the device in a first chamber. When a frangible seal of the first chamber is ruptured, the active compound is released to a second chamber, from which it is released through a permeable layer. That means, in WO2003/000088 a two stage release from two serial chambers is described. However, the device is not suitable for a compound that needs to be stored dry, oxygen free, or dark, and which is to be activated by moisture or by means of a fluid. The compound stored in the device of WO2003/000088 is already in activated condition during storage, and only released in a defined manner. Furthermore, nothing is mentioned of a gaseous active compound.

WO 2006/100155 A1 (NOLabs AB) relates to a device for treating wounds comprising a NO-eluting polymer. WO 2006/100155 teaches a patch with NO elution in only one direction, with a NO-impermeable material on one side and a NO-permeable membrane on the other side of the device. However, this device permits only a short-time-release of NO, requiring a frequent change with considerable stress for skin and patient.

In sum, application of known compounds generating NO for therapeutical treatments has been impractically complicated and time consuming up to now. In addition, many of the NO generating systems are very sensitive, e.g. to humidity, oxygen, and light, and have to be stored under special conditions. This makes it very difficult to integrate the NO donors in products that have a shelf life allowing advantageous industrial applicability.

Moreover, known devices show a number of disadvantages with regard to topical or transdermal delivery of NO from an NO donor.

Hence, an improved system or device for topical application of NO would be advantageous, and in particular a topical dermal delivery system allowing for instance one or more of increased flexibility, cost-effectiveness, storage time, convenience of use, patient friendliness, efficiency of therapy, and/or patient safety would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a topical patch, a dermal nitric oxide release system, a method of treatment, and a cosmetical method that allows for advantageous treatment, according to the appended patent claims.

According to a first aspect of the invention, a topical nitric oxide delivery device according to claim 1 is provided. The device comprises a backing layer substantially impermeable to nitric oxide, arranged and configured to limit a loss of nitric oxide concentration during release of nitric oxide in the delivery device; a nitric oxide permeable layer in use of the delivery device oriented towards an area to be treated; and an absorbing layer between the backing layer and said nitric oxide permeable layer, arranged to retain a nitric oxide releasing fluid therein, in such a manner that nitric oxide released from said nitric oxide releasing fluid is directed within the delivery device and through the nitric oxide permeable layer towards a skin area by an increase of partial pressure or concentration of nitric oxide therein. Furthermore, the backing layer comprises a sealingly reclosable, or closable opening, said opening comprises an activation window being closable by a backing lid, wherein fluid access into the device is provided by said opening or a fluid communication port.

According to a second aspect, a kit comprising a topical nitric oxide delivery device according to the first aspect of the invention is disclosed. The kit further comprises a separate container for an nitric oxide donor compound and an activation fluid, wherein said nitric oxide donor compound and said activation fluid are separated from each other a seal that is removable, such that said nitric oxide donor compound and said activation fluid are admixable prior to transferring to said device upon use thereof.

According to another aspect of the invention, a use of nitric oxide in the manufacture of a medicament according claim 16 is provided, that comprises a backing layer substantially impermeable to nitric oxide, arranged and configured to limit a loss of nitric oxide concentration during release of nitric oxide in the medicament; a nitric oxide permeable layer in use of the medicament oriented towards an area to be treated; and an absorbing layer between said backing layer and said nitric oxide permeable layer, arranged to retain a nitric oxide releasing fluid therein, in such a manner that nitric oxide released from said nitric oxide releasing fluid is directed within the delivery device and through the nitric oxide permeable layer towards a skin area by an increase of partial pressure or concentration of nitric oxide therein. Furthermore, the backing layer comprises a sealingly reclosable, or closable opening, said opening comprises an activation window being closable by a backing lid, wherein fluid access into the device is provided by said opening or a fluid communication port.

According to another aspect a method of topically delivering nitric oxide is disclosed. The method comprises activating nitric oxide release from a nitric oxide donor system in a nitric oxide delivery device according to the first aspect of the invention; placing the activated nitric oxide delivery device in contact with a skin area of a body, with a nitric oxide permeable layer of the delivery device oriented towards said skin area; and delivering nitric oxide topically from said nitric oxide delivery device to said skin area via said nitric oxide permeable layer, wherein a permeability of said nitric oxide permeable layer is selectively chosen depending on a toxicity level of said nitric oxide donor system.

According to a further aspect, a method of treatment is disclosed, and comprises delivering nitric oxide according to the above aspect of the invention to a therapeutic treatment site, such as a site of peripheral neuropathy, diabetic ulcers, wounds, skin infection, fungal dermal attack such as onychomycosis, mixed dermal infections, and/or virus attack such as warts.

According to a further aspect of the invention, a cosmetical method is provided. The cosmetical method comprises delivering nitric oxide according to the above aspect of the invention to a cosmetic treatment site, such as a site of scars to be reduced by said nitric oxide, or a site of sagged skin or wrinkles to be lifted by said nitric oxide.

Features for the second and subsequent aspects of the invention are as for the first aspect mutatis mutandis.

Further embodiments of the invention are defined in the dependent claims.

Some embodiments of the invention provide for patient friendly, convenient delivery of nitric oxide to a skin area.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic illustration of a topical patch that has a multi layered structure,
Figs. 2A to 2D are cross sectional views of embodiments of patches having various NO permeable layers;
Figs. 3A to 3C are cross sectional views of embodiments of further patches having various arrangements of an NO impermeable backing layer;
Fig. 4A is a perspective view of a patch having a sealingly closable backing enabling fluid access to the patch interior during activation;
Fig. 4B is a cross sectional view of the patch shown in Fig. 4A;
Fig. 5 is a schematic illustration of a topical patch that has a multi layered structure;
Fig. 6A is a perspective view of a patch having an access port to the interior of the patch system;
Fig. 6B is a cross sectional view of the patch shown in Fig. 6A;
Fig. 7A is a perspective view of a patch having a sealingly reclosable backing enabling exchange of a patch system component during use;
Fig. 7B is a cross sectional view of the patch shown in Fig. 7A as well as a further patch system component comprising an exchange component of the patch system;
Fig. 8 is a schematic illustration of a patch system component comprising an NO donor component and an activation fluid in a breakable receptacle;
Fig. 9 is a schematic illustration of a patch system component comprising an NO donor component and an activation agent in a multiple compartment arrangement;
Fig. 10 is a schematic illustration of a patch system component comprising an NO donor component and an activation agent in a multiple compartment arrangement of a bottle;
Fig. 11A is a perspective view of a patch system comprising a patch and the patch system component of Fig. 9 prior to admixing the NO donor component and a release activation fluid;
Fig. 11B is a perspective view of the patch system of Fig. 11A after admixing the NO donor component and a release activation fluid and filling the patch with the activated mixture;
Fig. 12A is a perspective view of a further patch system comprising an NO donor component and an activation agent in a multiple compartment arrangement;
Fig. 12B is a cross sectional view of the patch system shown in Fig. 12A along a line A-A;
Fig. 13 is a perspective view of a further patch 15 system comprising an NO donor component and an activation agent in a breakable multiple compartment arrangement;
Fig. 14 is a perspective view of a patch having multiple compartments; and
Fig. 15 is a perspective view of a patch having passageways for a fluid to and from the skin to which the patch is applied in use.

### Description of embodiments

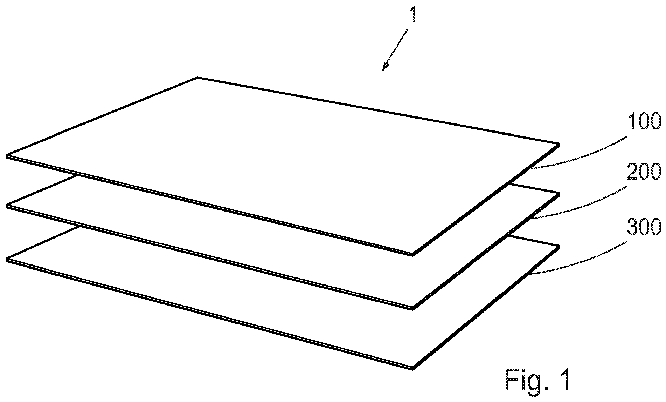

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.
The following description focuses on an embodiment of the present invention applicable to a topical patch, and in particular to a topical patch releasing NO. However, it will be appreciated that the invention is not limited to this application but may be applied to many other delivery devices, including for example wraps, bandages, etc. As illustrated in Fig. 1, embodiments of the topical patch 1 have a layered structure comprising an NO permeable layer 300, such as a membrane, in use of the patch oriented towards the area to be treated, and an NO non-permeable layer 100, i.e. a layer that is substantially not permeable for NO, such as a suitable impermeable membrane or film, as a backing. Between these two layers, an absorbing or reservoir layer 200 is provided, also called an absorbing core. The absorbing core may be provided in the form of a fluid retention system in order to minimize leakage and to provide a reservoir for an NO donor.

Further below, a number of embodiments will be given with regard to each of the NO permeable layer 300, the absorbing layer 200, and the non NO permeable layer 100. Furthermore, additional layers being arranged adjacent to these layers will be described in connection with some embodiments.

When activating release or elution of NO from the NO donor, or more specifically from an NO donating compound, provided at the absorbing core, the released NO diffuses within the patch 1. Some embodiments of suitable NO compounds are given further below. No may be provided in gaseous form or dissolved in a liquid. Since the direction of the diffusion is controlled by the principle of minimum diffusion resistance, i.e. diffusion takes place in the direction of the least hinder, NO only escapes substantially through the NO permeable layer 300. The NO permeable layer 300 is for instance a membrane selectively permeable for gaseous NO or dissolved NO. Thus, in use of the patch, the NO released inside the patch, is directed towards the skin of the patch user through the NO permeable membrane. The NO release is effectively targeted towards the treatment site.

### NO non-permeable layer 100

In addition, the non NO permeable layer 100, during use oriented away from the patient, i.e. on the outer side of the patch, does not only contribute to directing the released NO towards the skin, it also contributes to increasing the partial pressure of NO inside the patch and therefore increases the concentration of dissolved NO therein. The NO non-permeable layer 100, such as a membrane non-permeable for NO, serving as a backing layer, is arranged and configured to limit the loss of concentration during the production of NO in the patch as well as see to that the NO concentration or partial pressure increases, at least during the initial phase of NO release. In such a manner, the backing layer may for instance assure an increased amount of dissolved NO within the patch. In addition backing layer 100 may comprise inlet ports, such as Luer lock ports for access to the interior of the patch.

### Absorbing layer 200

The absorbing layer 200, i.e. the absorbing core, may have multiple functions during the use of the patch. It stores and retains an activation fluid, activating the release of NO from an NO donor compound. Furthermore, it also interacts with the NO donor compound to ensure minimal migration of the donor out from the patch. In the case of precipitation of the NO donor after NO release, the absorbent efficiently traps and contains such possible residues. Another positive property of the absorbent system is that it ensures that the NO donor has continuous contact with the activation system and thus ensures a continuous NO release.

In an embodiment, the absorbent core is provided in form of an absorbing system, e.g. a gel based system generated by a super absorbent polymer, such as super absorbent powder (SAP) and/or super absorbent fibers (SAF) or equal gelling material, that absorbs and retains the fluid. The absorbing component may be incorporated with other materials known to the skilled person, e.g. for providing wicking, mechanical strength, etc. In order to enhance shelf life of the patch, the NO donor compound is not activated for NO release prior to use. That means, even if the NO donor compound is arranged in a non-activated form, the above described gel based system is activated during storage. It is not feasible to maintain an NO donor compound in a gel form for long-term storage, prior to use, in a patch. When providing drugs, according to the prior art, in gels as well as different reservoirs, this is done for permanent storage of the drug in the reservoir focusing on containing it in a stable form. This stands in contrast to the required targeted activation of NO release upon use of the patch. The reservoir in the present application has a function during the actual NO release and in that case being a storage and entrapment for both the activation fluid as well as the donor in a reservoir that contains these substances in a fluid stage. The fluid stage may be a gel structure generated by a super absorbent polymer SAP/SAF or equal gelling material that contains the fluid within its boundaries.

Advantageous NO transport from the patch is provided when the absorbent core is in good contact with the membrane that is NO permeable towards the area to be treated. In this way good NO transport to the treatment site is ensured. One way to obtain this is to have the absorbent close to full wettening so that the fluid trapped in the system comes in close contact with the membrane. Full wettening is achieved in dependence on the absorbent system and/or the NO donor system, and may be achieved at rates exceeding 60% fluid content thereof, e.g. more than 80% or more than 90%. Another way is to glue the membrane on the absorbent.

The above-mentioned NO reservoir system based on an absorbent core, assures that NO release from the patch is maintained throughout the whole treatment and that both the activation fluid and the NO donor are safely contained within the patch.

Thanks to the advantageous NO production in the patch and the use of a gel, it is assured that NO that is generated in a liquid stage and is maintained in that stage before diffusing towards the treatment site. Furthermore, the NO donor compound itself, which may be toxic, is contained in the patch, avoiding that the NO donor comes into contact with the skin. In addition, when the NO donor in specific cases precipitates after or during NO release, any residues are trapped, and can not come into contact with the skin.

Applicants have found that the efficiency of a topical patch is increased with dissolved NO in comparison to gaseous NO. The backing contributes to increasing the partial pressure of NO inside the patch and therefore increases the concentration of dissolved NO therein which is more efficient than gaseous NO. The distribution of NO donor prior to activation also increase the possibility of high NO concentration due to minimizing local over saturation and thus minimizing NO gas formation. Furthermore, NO release from the patch is most effective at a fluid content relationship of the absorbent core at more than 60% fluid content thereof, e.g. more than 80% or more than 90%, depending on the fluid contact with the NO permeable membrane.

Pure gas treatments, e.g. as described in US2004/0009238, require very high concentrations of NO in the delivery gas, e.g. 200ppm or more. Such high concentrations could be dangerous for the user, if for instance inhaled by mistake.

Other suitable reservoir systems might be provided with some embodiments, i.e. not only an absorbent core of a matrix material. Alternatively, also a fluid, viscous fluid or gel may be provided in some embodiments.

### NO donor systems

Some embodiments of NO donor systems for generating NO in the patch may be based on compounds comprising polymers comprising an 0-nitrosylated group, such as diazeniumdiolate groups, S-nitrosylated and 0-nitrosylated groups, or any combinations thereof.

The nitric oxide (NO) eluting polymer may comprises diazeniumdiolate groups, S-nitrosylated groups, and 0-nitrosylated groups, or any combination of these.

Some embodiments of NO donor systems for generating NO in the patch may be based on NONOates.

Some embodiment may be provided with a nitrite/acid system for delivery of NO.

Activation fluid includes proton donors and comprise without limitation liquids such as water, physiological buffers, and saline. The activation fluid activates release of nitric oxide from an nitric oxide donor, like the aforementioned compounds, in the NO delivery device 1 for topical delivery.

### NO permeable layer 300

The NO permeable layer 300, such as a membrane, in use of the patch oriented towards the area to be treated, is devised to regulate the diffusion of NO, e.g. dissolved or gaseous NO, out of the patch towards the skin. Thus, the NO permeable layer 300 is configured to regulate the NO transfer speed from the patch to the user's skin.

Furthermore, the membrane may be chosen in dependence on the NO generating system in the patch. For instance, the NO generating system may comprise NO donor compounds or degradation products that are toxic, irritant, or non-toxic. The NO permeable layer 300 may be chosen in dependence of this level of skin compatibility, i.e. different membranes may be suitable. In this manner, the patch may be provided in a cost-efficiently manner while maintaining patient comfort.

In the case of good communication allowed across the NO permeable layer 300, e.g. when the NO generating system is non-toxic, the NO permeable layer 300 may be made in form of a membrane of nonwoven or porous materials that permit fluid transport. In this case, the NO released from the NO generating system may be transported dissolved in the fluid across the membrane to the skin.

In the case of medium communication is allowed across the NO permeable layer 300, e.g. when the NO generating system is irritant, and in case the activation fluid is water, a high moisture vapor transfer rate (MVTR) material, such as a micro porous membrane or a monolithic partially hydrophilic block polymer membrane, may be provided in some embodiments. Such membranes hydrophilic parts will quickly be filled with water and the NO may be transported dissolved in the water across the membrane. In the case of low communication is allowed across the NO permeable layer 300, e.g. when the NO generating system is moderately toxic, it is necessary to hinder medium to large components from traveling across the NO permeable layer 300. In case the fluid is water, a medium/low MVTR material is chosen that is slowly be filled with water. NO is mainly transported together with the water.

In the case no communication is allowed across the NO permeable layer 300, e.g. when the NO generating system is toxic, it is necessary to totally separate the interior of the patch system from the skin with regard to the NO generating systems. The material of the NO permeable layer 300 is chosen such that no liquid communication is allowed across the membrane. Any liquid transport is fully prevented by the membrane. In case the fluid is water, at least one layer is provided in the membrane, which is hydrophobic, in order to hinder water transport across the membrane. In this case, NO is transported up to the hydrophobic layer dissolved in the water. From the hydrophobic layer, withholding the fluid water, NO diffuses from the water through the hydrophobic layer towards the skin.

Suitable membrane materials are Silicone, eo-polyester, EVA, PTFE, as well as PUR that has sufficient permeability to be used, depending on configuration and material with regard to providing selectively fluid or water permeation or not.

Hitherto membranes have been permeable for substances that are made of much larger molecules than NO. NO permeable layer 300 is configured to be selectively permeable for nitric oxide molecules. In this manner the NO permeable layer 300 provides for an efficient protection barrier preventing unwanted NO donor compound components, to be transferred via the NO permeable layer 300 during the transfer of NO.

Hence, the NO permeable layer 300 not only regulates the NO release from the patch, is also ensures that the NO donor compound or components thereof, are only exposed to the skin if allowed, e.g. when non-toxic or non-irritant. US2005142218 of Benjamin et.al discloses a barrier consisting of a membrane that allows diffusion of non-gaseous nitrate ions while preventing direct contact of the skin and an acidifying agent. The membrane disclosed in US2005142218 comprises a pharmacologically acceptable acidifying agent and a pharmacologically acceptable source of nitrite ions or a nitrite precursor therefore. The membrane may be fully-, or partially-permeable, including semi-permeable or selectively permeable, to the passage of nitric oxide ions. Such membranes can prevent direct contact of the composition with the skin but can permit diffusion of nitric oxides into the skin. This means that the membrane disclosed in US2005142218 is not selectively permeable for NO, and does not control that an NO donor compound or components thereof, are not exposed to the skin. Furthermore, directed, efficient application of NO to a skin site is not disclosed. Moreover, fluid access into the patch is not provided.

A further positive feature of embodiments of the NO permeable layer 300, such as membrane, is that transport of NO donor compounds through the membrane is minimized.

### Skin Contact Enhancer

The NO permeable layer 300, in use of the patch oriented towards the skin of the patch user, may comprise a skin contact enhancement layer or have arranged an adjacent skin contact enhancement layer, or it may have such contact enhancement properties that it provides sufficient skin contact properties by its intrinsic suitable properties.

The contact to the skin can be enhanced by an adhesive layer, such as a silicone gel layer, a pressure sensitive adhesive (PSA), or a hydrogel layer. The skin contact enhancement layer provides good contact between the membrane and the skin. In embodiments, the adhesive layer is continuous and is configured to fill out unevenness in the skin. Furthermore, the skin contact enhancement layer provides enough adhesion capabilities to stick the patch to the skin.

The skin contact enhancement layer has to be permeable for NO. Both a good skin contact of the patch and a sufficient transport of NO through the skin contact enhancement layer are provided by the patch according to the embodiments described herein. Previously, it has not been not taken into account that the NO transport through such a skin contact layer is an issue. The skin enhancement layer needs to be sufficiently permeable for NO in order to provide a patient friendly and effective release of NO from the patch to the skin of the user. This issue may be resolved by attaching the skin contact layer to the NO permeable membrane on the side that in use is turned towards the skin. Further below, suitable materials and other features for an optimal NO transport through an NO permeable layer 300 and/or a skin contact enhancement are described in more detail.

Some embodiments comprise a skin contact layer that may be used repeatedly for continuous therapy, wherein such embodiments comprise an absorbent exchange system that may be replaced after a certain time of use with a fresh one without removing the patch from the skin of the user.

### Fluid direction system

Some embodiments of the patch may comprise a fluid direction system including an NO donor compound. The fluid direction system is arranged to receive an activation fluid and direct the activation fluid to an NO donor compound, whereupon the activation fluid dissolve the NO donor compound prior to applying it into the absorbent core. Some embodiments comprise a fluid direction system in form of a spread systems as well as connection points to the patch for receiving the activation fluid. For instance, a single injection point for introducing the activation fluid into a sealed patch is provided. In the patch the fluid is absorbed as described.

### Storage of the patch

In some embodiments, the challenge to ensure safe long term storage of the patch system, or at least of the NO donor compound thereof, is solved by individual storage of the different components. By storing the sensitive NO donor compound in a separate compartment within or separate from the device, it is ensured that the necessary parameters are fulfilled. The environmental parameters that may affect the NO donors are for example moisture, oxygen, and light. On the other hand, the separation of some individual components of the patch system puts high requirements on the design so that the integration of the components may be performed in simple, user-friendly, reliably and efficient manner. According to the invention, this challenge is solved by means of a sealingly reclosable, or closable opening, such as an activation window, provided in the backing of the patch, i.e. in the NO non-permeable layer of the patch. Such an activation window enables an exchange of at least a part of the absorbent core, for instance when the NO release has decreased. This provides to sustain the NO release from the patch over a long period of time without changing the membrane towards the skin, which is an advantage for the user as the skin is less stressed by removing the patch less frequently from the skin.

In addition, fluid access into the device is provided according to some embodiments, e.g. by a fluid communication port. This allows a patch to be stored for a long term, e.g. under dry, oxygen free and/or dark conditions, whereupon activation of NO release from the patch is provided via introducing a suitable fluid into the patch via such a fluid communication port.

Some specific embodiments will now be described in more detail.

### Topical patch

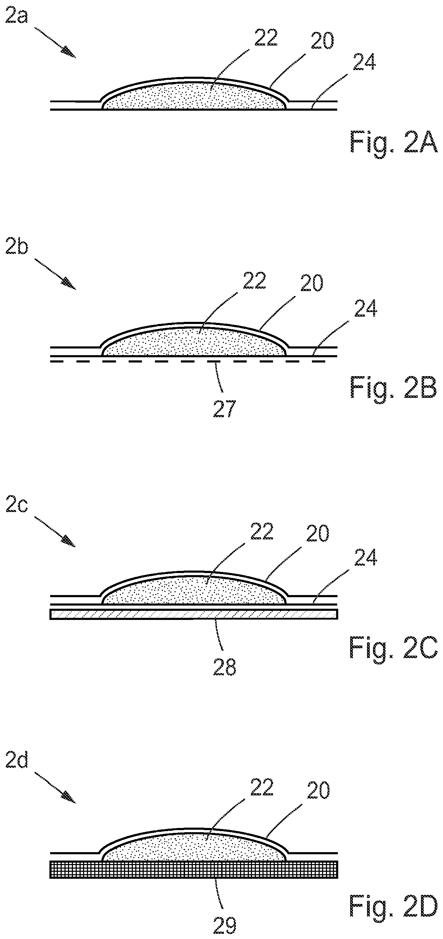

Topical patch systems for therapeutic or cosmetic treatment with nitric oxide is provided in a number of embodiments, as illustrated in Figs. 2A-2D, 3A-3C, 4A and 4B, 6A and 6B, 7A and 7B, 11A and 11B. Details of these embodiments are illustrated by means of the remaining Figs.

Figs. 2 A to 2D (not according to the invention) are cross sectional views of embodiments of patches 2a-2d having a fixed backing layer 20 and an absorbent core or reservoir 22 from which in use NO is provided for treatment. Patches 2a-2d have different arrangements on the patient side of the patch. Patches 2a, 2b, and 2c comprise a NO permeable membrane layer 24. Patch 2d comprises an NO permeable gel 29. Patch 2b comprises a pressure sensitive adhesive (PSA) skin contact enhancement layer. Patch 2c comprises a silicone gel layer 28 serving as a skin contact enhancement layer. Patch 2d comprises a gel membrane 29.

The layers of patches 2a-2d are shown apart for illustrative purposes only. Assembled patches 2a-2d are provided with the layers in close contact, in such a manner that the function of efficient topical NO application is provided. Patches 2a-2d also comprise adhesive joints or layers, which are not illustrated, in order to suitably attach the layers to each other. For instance, backing layer 20 is glued to membrane 24 around the absorbent core or reservoir 22. Backing layer 20 may also be glued to the absorbent core or reservoir 22.

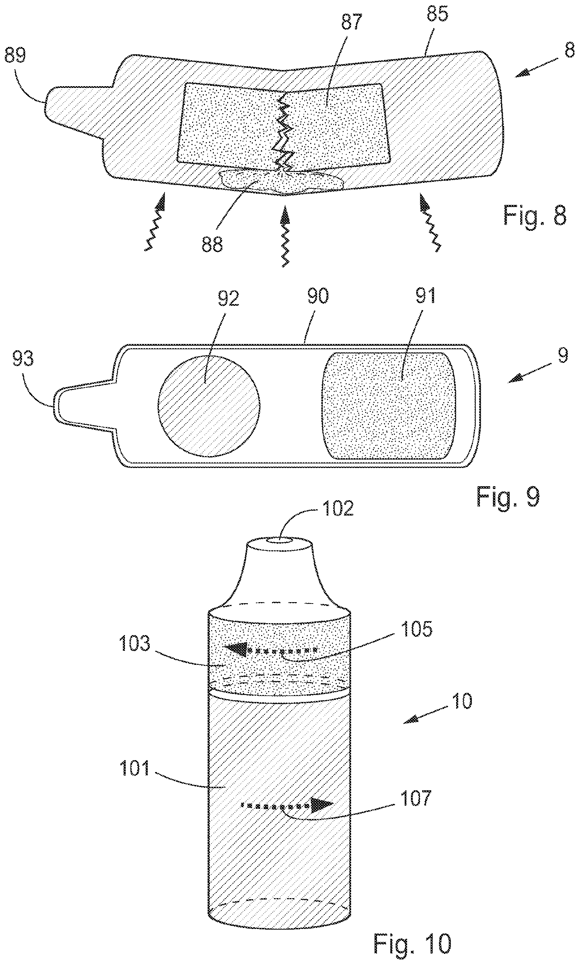

Patches 2a-2d comprise both an NO donor compound and an activation system suitably separated from each other during storage. Suitable ways of separation are described further below, for instance with reference to Figs. 8, 9 or 12, e.g. by having the NO donor in a glass ampoule that is broken upon use in order to create a contact of the NO donor and an activation fluid, e.g. a SAP/SAF absorbent core that is saturated with more than 90% water.

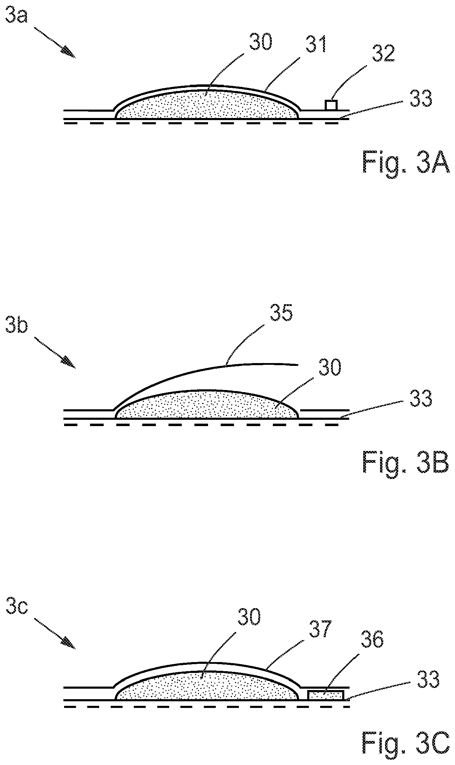

Figs. 3A to 3C are cross sectional views of embodiments of further patches, wherein variations of the absorbent core or reservoir, or of the backing layer are illustrated. (Figs. 3A and 3C are not according to the invention). Patches 3a-3c are illustrated having an NO permeable membrane layer 33. As indicated by the dashed line in Figs. 3A-3D, patches 3a-3c may also comprise additional or alternative skin enhancement layers, or combined skin enhancement layers and membranes, such as the ones illustrated in Figs. 2A-2D, for instance a permeable gel membrane etc. As in Figs. 2A-2D, the layers of patches 3a-3c are shown apart for illustrative purposes only. The patches 3a and 3b have a backing layer 31, 35, respectively, which provide access to the absorbent core or reservoir 30 from which in use NO is provided for treatment. Patches 3a-3c may have an NO donor compound incorporated into the absorbent core or reservoir 30. As access is provided to the interior of the patch, an activation fluid may be introduced into the interior of the patch for activation of NO release from the NO donor compound. Patch 3a is provided with an access port 32 in an impermeable backing 31 to a pouch in the interior of patch 3a, comprising the absorbent core or reservoir 30. Through port 32 communication is given with the interior of patch 3a. Port 32 is equipped with suitable units ensuring that fluid only enters the patch and leakage is avoided, such as one way flap valves. Through port 32 an activation fluid may be introduced into patch 3a for activation of release of No from an NO donor contained therein, e.g. incorporated into an absorbent core. An activated, pre-prepared NO releasing fluid may also be introduced through port 32 into patch 3c for NO release therefrom. Patch 3b comprises a backing layer 35, which provides access to the absorbent core or reservoir 30. In this way, like through port 32, either an activation fluid or an activated NO releasing fluid may be introduced into the patch 3b. Backing layer 35 is sealingly closed after introducing the fluid into the patch 3b. A suitable adhesive, e.g. underneath a release liner that is removed prior to closing the lid, is provided on the inner surface of the flap of backing layer 35, such that an NO impermeable backing is provided after closing the lid. Patch 3c comprises a fixed backing, and a system of separated NO donor compound and activation fluid, which is arranged such that the NO donor compound and the activation fluid may be brought into contact by outer action, e.g. by removing an intermediate impermeable membrane. Further details for this type of patch are given below.

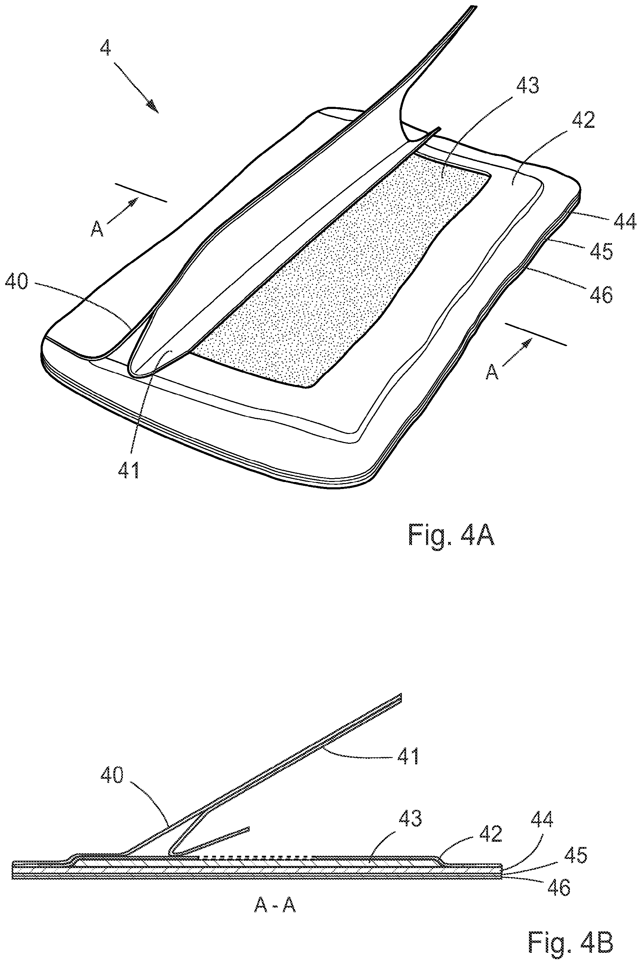

Fig. 4A is a perspective view of a patch 4 having a closable backing layer 40, enabling fluid access to the patch interior during preparation of the patch 4 for use. Fig. 4B is a cross sectional view of the patch 4 illustrating the arrangement of the various layers and components of the patch 4 prior to application on the skin.

An access window to an absorbent core or reservoir 43 that is accessible from the exterior prior to use of the patch 4. The access window is arranged in an intermediate layer 42 of the same material and characteristics as backing layer 40, i.e. NO impermeable and flexible. Upon use, either an activation fluid or an activated NO releasing fluid is introduced into the interior of patch 4, depending on if an NO donor compound is provided in the absorbent core or reservoir 43 or not. The closable backing layer is closed when NO release is initiated in the aforementioned way. A release liner 41 is arranged releasably on backing layer 40 and removed for adhesively closing the window by means of backing layer 40 being attached to intermediate layer 42. Thus an NO impermeable arrangement is provided on the backing side of patch 4. Thereupon a second release liner 46 is removed from a skin contact enhancing layer 45, and the patch 4 is applied to the skin, whereupon NO is provided to the skin from the patch 4.

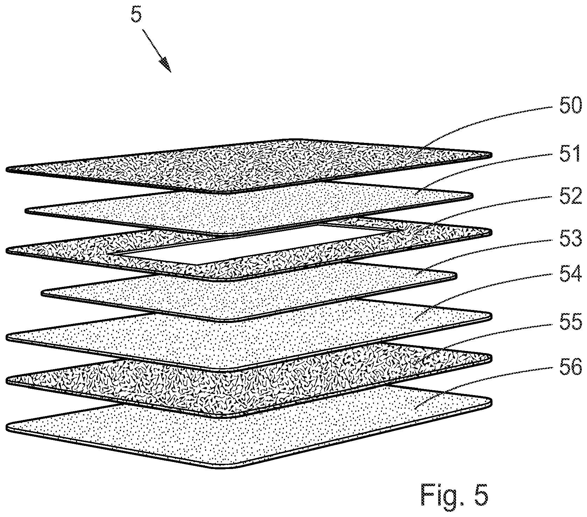

Fig. 5 is a is a schematic illustration of the multi layered structure 5 of the embodiment of Fig. 4A. An NO impermeable backing lid 50 is arranged on top of the patch. A release liner 51 is arranged removably on backing lid 50. A windowed backing 52 is arranged on top of an absorbent layer 53, underneath which an NO permeable membrane 54 is arranged, followed by a skin contact enhancement layer 55 and a release liner 56.

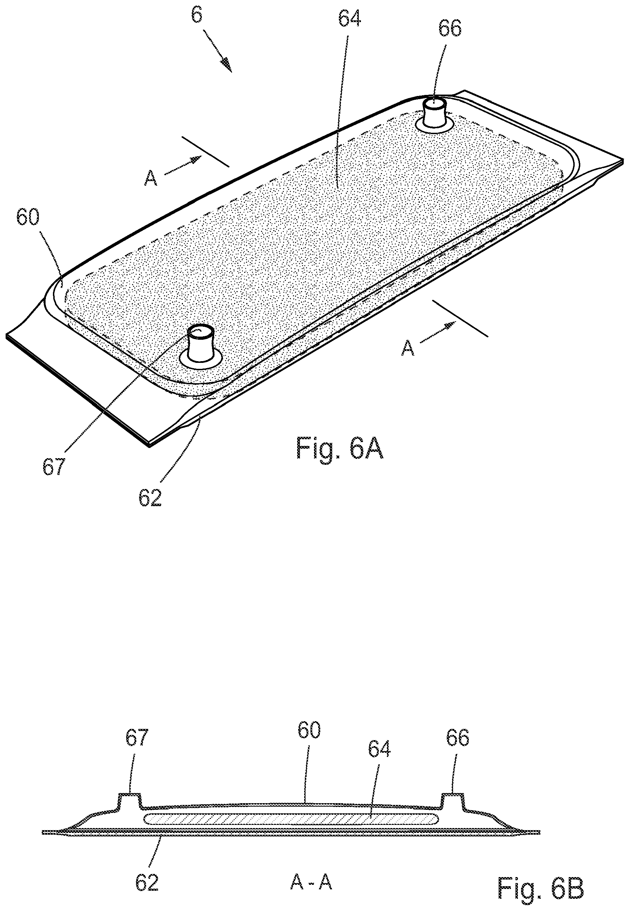

Fig. 6A (not according to the invention) is a perspective view of a patch 6 having two access port ports 66, 67 allowing fluid communication with the interior of the patch 6. Fig. 6B is a cross sectional view of the patch shown in Fig. 6A. A backing layer 60 is impermeable for NO and a NO permeable layer 62 is arranged on the patient side of the patch 6. An absorbent core or reservoir 64 is arranged inside the patch, for receiving a fluid via port 66 and/or port 67. The absorbent core or reservoir may be made of an airlaid material. Ports 66, 67 may be in form of a Luer lock system, e.g. for convenient connection of a syringe. A fluid guiding system may be arranged in, or adjacent to, the absorbent core or reservoir 64 for controlling distribution of the fluid in the patch 6.

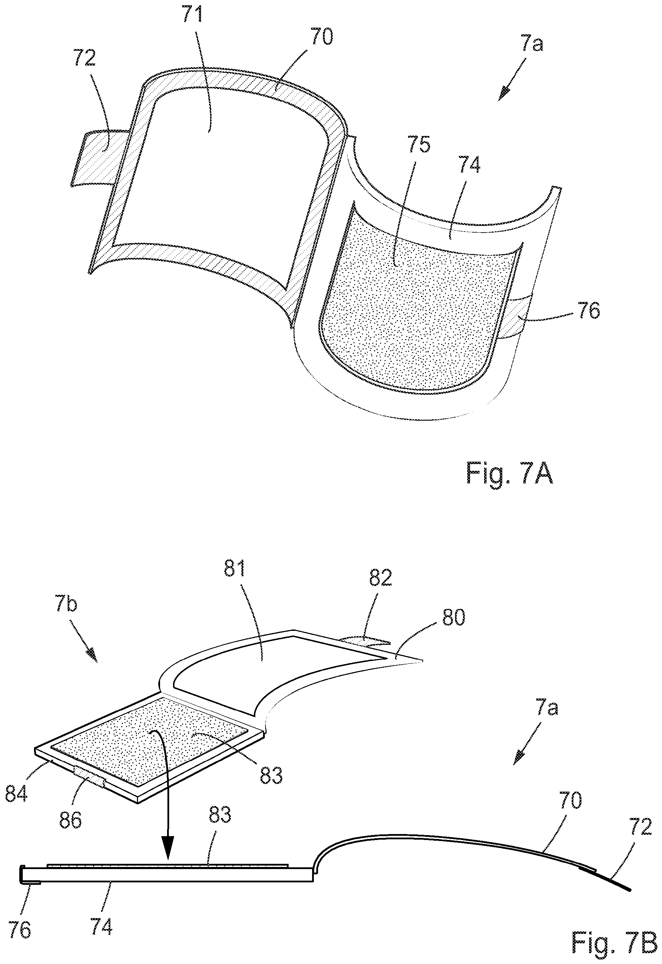

Fig. 7A is a perspective view of a patch 7a of a patch system. Fig. 7B is a cross sectional view of the patch 7a as well as a further patch system component 7b comprising an exchange component of the patch system. The patch 7a comprises a sealingly repeatedly open-and closable backing enabling exchange of a patch system component during use. More precisely, is the patch 7a provided with a recess 75 into which an exchangeable absorbent core or reservoir 83 may be removably positioned, as illustrated in Fig. 7B. An NO impermeable backing layer 71 comprises a secure gas tight releasable adhesive 70 around its edge for mating with an edge 74 of or around a NO permeable layer. For further security and patient comfort and user friendliness, a loop and hook fastener 72, 76 is provided on the patch 7a. Patch system component 7b is a package in which an NO donor compound is provided in form of an exchangeable component 83. The package comprises a top layer 80. Top layer 80 is during storage in a gas tight manner attached to a bottom layer 84 in which the exchangeable component 83 is situated. Both top layer 80 and bottom layer 84 are NO impermeable and adapted for long term storage of the NO donor compound in exchangeable component 83, e.g. gas tight, evacuated, and not transmissible for light. A closure system 82, 86 is provided on the packaging 7b. The exchangeable component 83 fits into the recess 75 in patch 7a. The exchangeable component 83 may be replaced in patch 7a with a fresh one, in order to recharge patch 7a with new NO donor. The release of NO from the NO donor is activated by adding fluid to the exchangeable component 83 either in the packaging 7b, or in the patch 7a. Then the backing lid is re-closed in a manner impermeable for NO, and the patch 7a is ready for continued use.

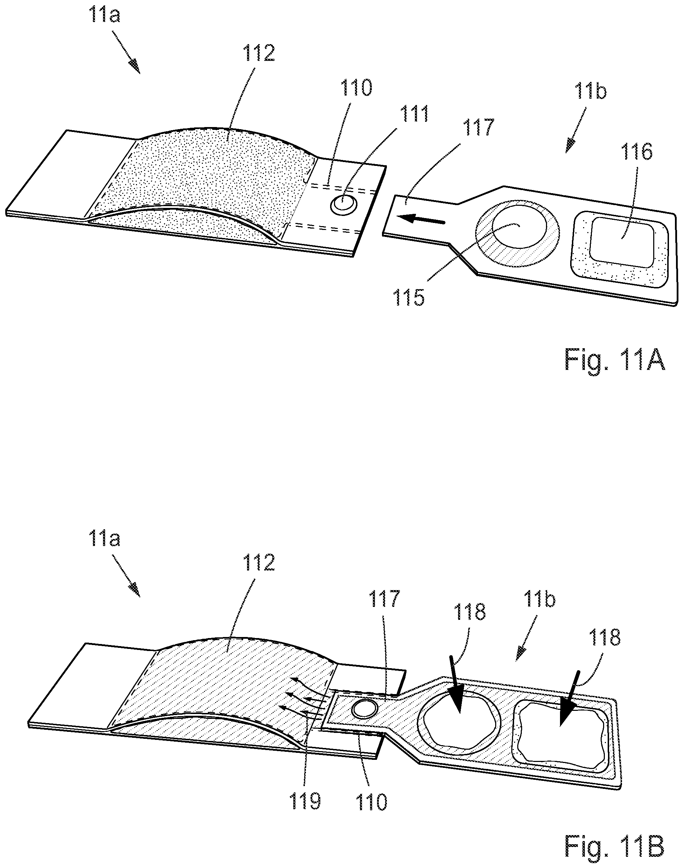

Fig. 8 (not according to the invention) is a schematic illustration of a patch system component in form of a sealed bag 8, comprising an NO donor 88 in a breakable receptacle 87. By external force the receptacle 87, e.g. a glass vial, is broken and the NO donor is released into the surrounding activation fluid in the bag 8. Thus, the NO donor is activated and NO release is initiated. Via an outlet 89, the activated fluid may be applied to a patch, such as the patch shown in Fig. 4A or Fig. 11A.

Fig. 9 (not according to the invention) is a schematic illustration of a patch system component comprising an NO donor component 92 and an activation fluid 91 in separate compartments of a multiple compartment device 9. Inside housing 90 of the device 9 the compartments may be broken, e.g. by external pressure on the compartments. The NO donor component 92 and activation fluid 91 are released from their compartments into a surrounding mixing chamber inside housing 90. Mixing may be enhanced by shaking the device 9. The fluid that thus is activated for NO release from the NO donor, may be transferred to a patch via an outlet port 93.

Fig. 10 (not according to the invention) is a schematic illustration of a patch system component comprising an NO donor component 103 and an activation agent 101 in a multiple compartment arrangement of a bottle 10. The bottle is arranged such that the two compartments are twistable against each other, where upon a seal between the two compartments is broken and the NO donor component 103 and the activation agent 101 get into fluid contact in the bottle 10. Mixing may be enhanced by shaking the bottle 10. The fluid that thus is activated for NO release from the NO donor, may be transferred from the bottle to a patch via an outlet port 102.

Fig. 11A (not according to the invention) is a perspective view of a patch system comprising a patch 11a and the multiple compartment device 11b of the type illustrated in Fig. 9, prior to admixing the NO donor component and the release activation fluid from two compartments 115, 116. The arrow at the outlet port 117 illustrates how the multiple compartment device 11b may be joined with an inlet port 110 of the patch 11a, as illustrated in Fig. 11B. Fig. 11B is a perspective view of the patch system of Fig. 11A after admixing the NO donor component and the release activation fluid in multiple compartment device 11b, as explained above. The activated NO releasing fluid is introduced into the patch and absorbent core or reservoir 112 via outlet port 117 of the multiple compartment device 11b and inlet port 110 of the patch 11a, wherein the flow is illustrated by arrows 119. During filling, leakage may be reduced or avoided by a valve 111, schematically illustrated in Figs. 11A and 11B. When the activated fluid is transferred to the patch 11a, the multiple compartment device 11b is removed, valve 111 is closed and the patch 11a is ready for application to the patient.

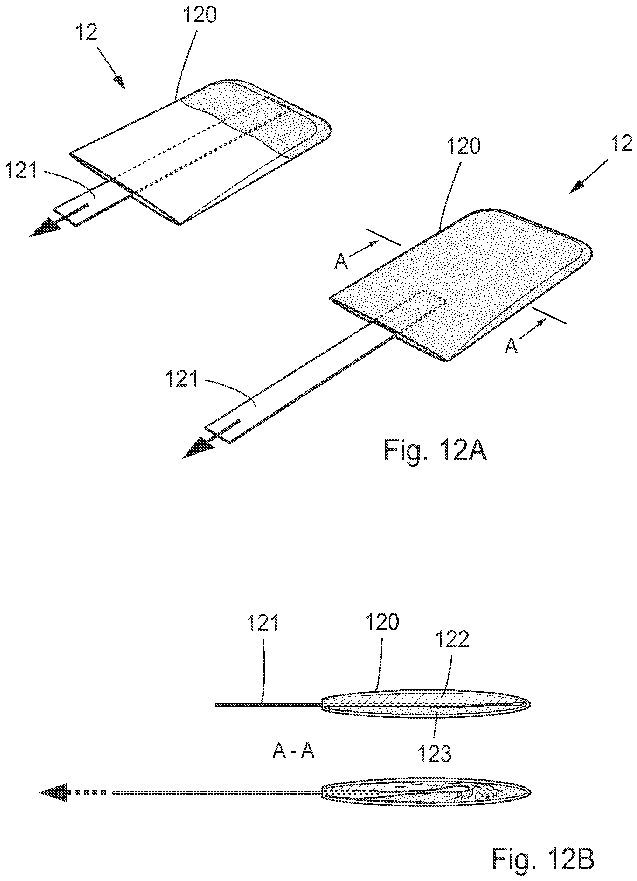

Fig. 12A (not according to the invention) is a perspective view of a further patch 12 comprising an NO donor component and an activation agent in a multiple compartment arrangement. Fig. 12B is a cross sectional view of the patch 12 shown in Fig. 12A along a line A-A. The patch 12 comprises a first compartment 122 and a second compartment 123, divided by an impermeable film, e.g. of aluminum foil. The first compartment 122 contains an NO donor compound and the second compartment 123 contains an activation fluid. The impermeable film may be removed between the compartments, as illustrated, by drawing a strip 121 attached to the impermeable film. In this manner, activation is easily accomplished for the user and the patch 12 is ready for application.

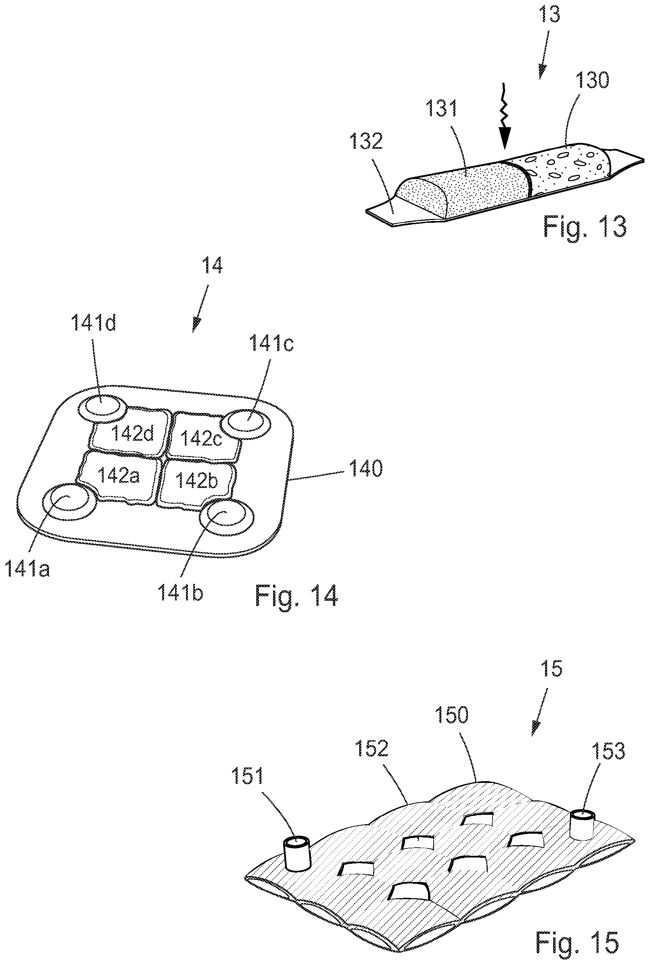

Fig. 13 (not according to the invention) is a perspective view of a further patch 13 comprising an NO donor component 131 and an activation agent 130 in a breakable multiple compartment arrangement 132. The two compartments may be connected to each other by breaking a seal that is arranged in-between by external force. A NO releasing fluid may thus be activated, wherein the patch 13 may also be shaken for intensely mixing the two components. A release line is then removed from an NO permeable membrane of patch 13 and the patch is ready for application.

Fig. 14 (not according to the invention) is a perspective view of a patch 14 having multiple compartments, subdividing the active area of the patch into different treatment zones corresponding to different compartments 142a-d. Each of compartments 142a-d is divided from the other compartment and accessible via a fluid communication port 141a-d, respectively. In this way the active usage time of the patch may be prolonged my sequentially filling the compartments with a NO releasing fluid or an activation fluid, depending on whether a compartment 142a-d contains an NO donor compound or not. The compartments may be geometrically arranged in a different way than illustrated, e.g. interleaved such that the treatment area is covered by each of the compartments. By means of this embodiment, the treatment time may be enhanced by activating the compartments subsequently when the useful amount of NO released from the NO donor compound decreases to a predefined threshold. This threshold may be indicated by the patch itself, e.g. by changing color at the surface of the compartment where NO release is below the threshold. The patch may comprise an active indicator on top of the patch, in order to indicate to the user that the NO has been utilized.

Fig. 15 (not acording to the invention) is a perspective view of a patch 15 having passageways 152 for a fluid to and from the skin to which the patch 15 is applied in use. The patch is built up of adjacent compartments 150 fluidly connected to each other and to two ports 151, 153. The patch 15 is filled with NO releasing fluid or activated as described above. When applied to a wound, the patch 15 allows for passage of exudate through the passageways 152, e.g. to an exchangeable absorbent applied on the back of patch 15.

The base of the topical NO delivery system is the absorbent core or reservoir in which the active mixture of NO donor and activation fluid is provided upon activation of the delivery system for use. This to provide an even spread of the donor over the device, to give a stable containment of the donor and to assure an even release during the treatment.

NO donor systems that comprise both a donor powder as well as an activation solution can in same cases be irritating to the skin and therefore a selective membrane can be placed in between the absorbent core and the skin.

A mechanical property of the NO delivery system components, such as the NO permeable membrane, is that they are flexible and therefore are able to adapt to the skin surface to assure good contact.

The NO releasing membrane material is selectively permeable for NO that is allowed to penetrate to the skin while passage of other components via the membrane is minimized.

In order to direct the NO release towards the treatment site, a top cover of a non-permeable material such as a thick layer of PE/ PUR may be used. This cover also increases the concentration of NO in the fluid since the partial pressure of NO in gas state is increased.

### Fluid system

Addition of fluids to the topical patch system may for instance be done in the following manners:
- By having a closable lid in the top cover through which all fluids can be pored in to the patch. The lid is then closed and the device can be attached to the skin, see Figs. 4A and 4B.
- By adding a one directional valve through which the fluids can be inserted in to the patch pouch, see Figs. 6A and 6B. In the case of integrating the NO donor compound in the pouch, all matter that may activate a release of NO from the NO donor compound, such as moisture, air, oxygen, in the pouch is extracted from the pouch during manufacturing thereof. Optionally, the pouch may be filled with an inert matter. After storage, and for activation of the patch, an activation fluids is injected through the valve into the pouch and absorbed into the absorbent core, whereupon NO release is activated and the patch is "active" for medical or cosmetical treatment. In order for the fluid to be spread evenly over the area a wicking layer may be advantageously provided.
- By having an integrated first reservoir for the NO donor in the patch, which is arranged in a sealed manner apart from the fluid. The fluid may be integrated together with the absorbent core or in a separate second reservoir. Prior to use the first and perhaps the second reservoir are manipulated to open, e.g. by squeezing the reservoir(s) to break. The NO donor spreads and comes in contact with the activation fluid and the NO release starts.
- By adding a pH adjusting feature to the absorbent core, or in that the absorbent core in itself has this capability, the fluid to be mixed with the donor may be inactive and only when the donor fluid comes in contact with the absorbent core the system is balanced to a suitable pH and the NO release is initiated.

For mixing of fluids, i.e. mixing of the activation fluid with the NO donor compound that for instance is provided in powder form, a number of embodiments are now described. Mixing is performed in such a manner that the NO donor compound is spread evenly in order to provide an efficient controlled release of NO from the patch. Various embodiments comprise, amongst others:
- The NO donor compound is provided in dry form and a pre dissolving fluid is mixed with the NO donor compound separately from the patch in order to dissolve the NO donor efficiently and to provide a pre dissolved NO donor fluid.
   ∘ The pre dissolved NO donor fluid is added to the patch, wherein a component inside the patch, e.g. the absorbent core, is soaked with a NO release activation fluid. Thereby an effective, controlled release of NO is activated in the patch.
   ∘ Alternatively, the pre dissolved NO donor fluid is added to a NO release activation fluid, or vice versa, whereby a NO releasing fluid is provided. The NO releasing fluid is added to the patch, i.e. to a component inside the patch, e.g. the absorbent core, which in this manner is soaked with the NO releasing fluid is. Thereby an effective, controlled release of NO is provided from the patch.
- The NO donor compound and solid activation components are provided in dry form in the patch, e.g. as fibers or a powder incorporated into the absorbent core. A fluid is added to the dry patch upon use of the patch, resulting in the solid activation components dissolving in the fluid and thus activating the release of NO from the NO donor compound.
- The NO donor compound is provided in dry form and mixed with a fluid comprising a pre dissolving and activation fluid, or only an activation fluid. The mixture, in which NO release from the NO donor compound is activated, is add to patch for providing NO release from the patch.

Further features of the topical patch

Some embodiments of the topical patch have enhanced functionality by comprising features, such as:
- An exchangeable absorbent material is provided, such that the layer towards the skin to be treated is stable during treatment and does not need to be replaced. This is an important feature in specific conditions within the area of wound care treatment.
- A wicking system for ease of spreading the fluid over the absorbent core is provided in the patch. Such a wicking system may for instance be provided in the form of a layer that is configured to spread a fluid in the patch before it passes into the absorbent.

- The patch may be provided in the form of a fluid pouch with a valve controlling fluid direction into the pouch and allow displaced gas to egress from the pouch.
- The pouch may be provided with different sections to assure that an even distribution of the fluid is obtained. In addition different treatment zones within the patch maybe provided by the different sections.
- The absorbent core may be made of various materials adapted to absorb fluid and contain it. Examples for such materials are super absorbing materials, for instance comprising SAP and/or SAF in it, or a hydrophilic foam, or a hydro gel.

The patch has three layers; a backing with a activation window, a absorbent core as a reservoir and an NO permeable membrane with an adhesive layer towards the skin. The NO donor is applied into the device as a solution prior to application through the activation window. The window is closed after activation and the lid and backing is non permeable for NO in order to increase the partial pressure of NO and thus the concentration of dissolved NO. The patch is fixated on the area to be treated with an adhesive layer that also assures sufficient contact throughout the treatment. The generated NO moves within the patch and can escape through the NO permeable membrane towards the area to be treated. Depending on therapy need the patch release curve differs and is chosen accordingly.

### Examples:

### Example 1: Intense dose patch

The patch has a high NO dose level such as between 0,01-100pmol/(cm2*min), such as 0,1 pmol/(cm2*min). It has a short duration of use, approximately ten minutes up to two hours, such as 1 hour use. To accomplish this intensity and lengths in dose curve a sufficient amount of NO donor has to be used such as 0,01-10 mg/cm2, such as 0,1 mg/cm2. Also a low pH in the activation fluid is needed, less than pH 6, such as pH 5 . The volume of buffer needed is dependant of the NO donor amount used. For a 0' 1 mg NO donor/cm2 patch, it is necessary to have 0,1-0,4 mL/cm2 depending on buffer strength. To ensure sufficient contact with skin a contact enhancement layer is used.

### Example 2: Extended dose patch

The patch have a medium NO dose level such as between 0,001-10pmol/(cm2*min), such as 0,05 pmol/(cm2*min). It has an extended duration of use of six to twelve hours, such as eight hour use. To accomplish this intensity and length in dose curve, a sufficient amount of NO donor has to be used, such as 0,01-10 mg/cm2, such as 0,2 mg/cm2. In addition, a neutral to slightly acidic pH in the activation fluid is needed, such as pH 7. The volume of buffer needed is dependant of the NO donor amount used. For a 0,2 mg NO donor/cm2 patch it is necessary to have 0,2-0,8 mL/cm2, depending on buffer strength. To ensure sufficient contact with skin a contact enhancement layer is used.

### Treatments and therapies that the patch is advantageous for are for instance:

treatment of peripheral neuropathy;
treatment of diabetic ulcers;
wound care, such as post operative healing support; infection prevention and treatment;
cosmetic use, including scar reduction, skin lifting etc.;
antifungal treatment, for instance of onychomycosis or mixed infections;
anti virus treatment, e.g. for wart treatment.

The patch is suitable for human or for veterinary medicine.

A kit may comprise several components according to the above description in an advantageous manner.

The patch may be regarded a medicament according to certain embodiments.

A use of nitric oxide may be provided for manufacturing a patch according to some embodiments.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

## Claims

1. A topical nitric oxide delivery device comprising a backing layer (100) substantially impermeable to nitric oxide, arranged and configured to limit a loss of nitric oxide concentration during release of nitric oxide in the delivery device;
a nitric oxide permeable layer (300) in use of the delivery device oriented towards an area to be treated, wherein said nitric oxide permeable layer (300) is a membrane permeable for nitric oxide;
a nitric oxide generating system comprising a nitric oxide releasing fluid, wherein said nitric oxide releasing fluid comprises a nitric oxide donor compound and an activation fluid, wherein said activation fluid is for activation of a release of nitric oxide from said nitric oxide donor compound comprised in said topical nitric oxide delivery device; and
an absorbing layer (200) between said backing layer (100) and said nitric oxide permeable layer (300), arranged to retain said nitric oxide releasing fluid therein,
in such a manner that nitric oxide released from said nitric oxide releasing fluid is directed within the delivery device and through the nitric oxide permeable layer (300) towards a skin area by an increase of partial pressure or concentration of nitric oxide therein;
wherein said backing layer (100) comprises a sealingly reclosable, or closable opening, said opening comprises an activation window being closable by a backing lid (35, 40, 50, 71),
and wherein fluid access into the device is provided by said opening or a fluid communication port.

2. The topical nitric oxide delivery device according to claim 1, wherein said backing layer (100) is devised to provide said increase of partial pressure or concentration of nitric oxide in said fluid in said topical nitric oxide delivery device.

3. The topical nitric oxide delivery device according to claim 1 or 2, wherein said absorbing layer (200) comprises a gel based absorbing system of a gelling material.

4. The topical nitric oxide delivery device according to any preceding claim, wherein said absorbing layer (200) is fully wettened.

5. The topical nitric oxide delivery device according to any preceding claim, wherein said nitric oxide permeable layer (300) is only permeable for nitric oxide.

6. The topical nitric oxide delivery device according to any preceding claim, wherein the device is configured to provide an nitric oxide dosage level between 0,01-100µmol/(cm2*min), such as 0,1 µmol/(cm2*min), or between 0,001-10 µmol/(cm2*min), such as 0,05 µmol/ (cm2*min).

7. The topical nitric oxide delivery device according to any preceding claim, wherein the device comprises an amount of an nitric oxide donor compound in the range of 0,01-10 mg/cm2, such as 0,1 mg/cm2, or 0,2 mg/cm2.

8. The topical nitric oxide delivery device according to any preceding claim, wherein the device comprises a package that is moisture free, oxygen free, and/or light intransmissible.

9. The topical nitric oxide delivery device according to any preceding claim, wherein said nitric oxide permeable layer (300) is made of a medium or low MVTR material, silicone, co-polyester, EVA, PTFE, or PUR that has sufficient permeability for nitric oxide.

10. The topical nitric oxide delivery device according to any of claims 4-9, wherein said absorbing layer (200) comprises a fluid content of more than 60%, such as 80% or more than 90%.

11. The topical nitric oxide delivery device according to any preceding claim, wherein said nitric oxide permeable layer (300) comprises a skin contact enhancement layer or is arranged adjacent to a skin contact enhancement layer, or has inherent skin contact enhancement properties, that is permeable for nitric oxide, such that it provides sufficient skin contact upon use.

12. The topical nitric oxide delivery device according to claim 11, wherein said skin contact enhancement layer is an adhesive layer, such as a silicone gel layer, a pressure sensitive adhesive (PSA), or a hydrogel layer, said enhancement layer provides good contact between the said nitric oxide permeable layer (300) and said skin area upon use.

13. The topical nitric oxide delivery device according to claim 1, comprising a fluid direction system that is arranged to receive the activation fluid and direct the activation fluid to the NO donor compound, the fluid direction system is in the form of a spread system and comprises at least one fluid connection port for receiving said activation fluid.

14. The topical nitric oxide delivery device according to claim 1, wherein the activation fluid is stored separate from the device prior to use.

15. The topical nitric oxide delivery device according to claim 1, wherein at least a part of said absorbent layer is exchangeable through said opening.

16. Use of nitric oxide in the manufacture of a medicament comprising a backing layer (100) substantially impermeable to nitric oxide, arranged and configured to limit a loss of nitric oxide concentration during release of nitric oxide in the medicament;
a nitric oxide permeable layer (300) in use of the medicament oriented towards an area to be treated, wherein said nitric oxide permeable layer (300) is a membrane permeable for nitric oxide;
a nitric oxide generating system comprising a nitric oxide releasing fluid, wherein said nitric oxide releasing fluid comprises a nitric oxide donor compound and an activation fluid; and
an absorbing layer (200) between said backing layer (100) and said nitric oxide permeable layer (300), arranged to retain said nitric oxide releasing fluid therein,
in such a manner that nitric oxide released from said nitric oxide releasing fluid is directed within the delivery device and through the nitric oxide selectively permeable layer (300) towards a skin area by an increase of partial pressure or concentration of nitric oxide therein;
wherein said backing layer (100) comprises a sealingly reclosable, or closable opening, said opening comprises an activation window being closable by a backing lid (35, 40, 50, 71),
and wherein fluid access into the device is provided by said opening or a fluid communication port.

17. A method of cosmetic treatment comprising delivering nitric oxide, said method comprising:
activating nitric oxide release from a nitric oxide donor system in a nitric oxide delivery device according to any of claims 1-15;
placing the activated nitric oxide delivery device in contact with a skin area of a body, with a nitric oxide permeable layer (300) of the delivery device oriented towards said skin area; and
delivering nitric oxide topically from said nitric oxide delivery device to said skin area via said nitric oxide permeable layer (300), wherein
a permeability of said nitric oxide permeable layer (300) is selectively chosen depending on a toxicity level of said nitric oxide donor system, wherein said skin area is a cosmetic treatment site, such as a site of scars to be reduced by said nitric oxide, or a site of sagged skin or wrinkles to be lifted by said nitric oxide.

18. The method according to claim 17, comprising releasing said nitric oxide from said nitric oxide donor system such that a partial pressure and thus a concentration of dissolved nitric oxide in said nitric oxide delivery device increases for said delivering of nitric oxide.

19. The method according to claim 18, comprising fixating said nitric oxide delivery device to said skin area by means of a skin contact enhancement layer permeable for nitric oxide.

20. The method according to claim 18, wherein said nitric oxide donor system comprises an absorbent unit interacting with a nitric oxide donor compound of said nitric oxide donor system, ensuring minimal migration of the nitric oxide donor compound out from the patch and trapping residues of said nitric oxide donor compound after precipitation therein.

## Patentansprüche

1. Topische Stickoxid-Liefervorrichtung, die Folgendes umfasst:
eine für Stickoxid im Wesentlichen undurchlässige Trägerschicht (100), die dafür ausgelegt und konfiguriert ist, einen Verlust der Stickoxidkonzentration während der Freisetzung des Stickoxids in der Liefervorrichtung zu begrenzen;
eine für Stickoxid durchlässige Schicht (300), die in Verwendung der Liefervorrichtung in Richtung eines zu behandelnden Bereichs orientiert ist, wobei die für Stickoxid durchlässige Schicht (300) eine für Stickoxid durchlässige Membran ist;
ein Stickoxid-Erzeugungssystem, das ein Stickoxid freisetzendes Fluid umfasst, wobei das Stickoxid freisetzende Fluid eine Stickoxid-Donorverbindung und ein Aktivierungsfluid umfasst, wobei das Aktivierungsfluid zur Aktivierung einer Freisetzung von Stickoxid aus der in der topischen Stickoxid-Liefervorrichtung enthaltenen Stickoxid-Donorverbindung dient; und
eine Absorptionsschicht (200) zwischen der Trägerschicht (100) und der für Stickoxid durchlässigen Schicht (300), die dafür ausgelegt ist, das Stickoxid freisetzende Fluid darin zu halten,
derart, dass aus dem Stickoxid freisetzenden Fluid freigesetztes Stickoxid innerhalb der Liefervorrichtung und durch die für Stickoxid durchlässige Schicht (300) durch eine Zunahme des Partialdrucks oder der Konzentration des Stickoxids darin in Richtung eines Hautbereichs gelenkt wird;
wobei die Trägerschicht (100) eine abdichtend wiederverschließbare oder eine verschließbare Öffnung umfasst, wobei die Öffnung ein Aktivierungsfenster umfasst, das durch einen Trägerschichtdeckel (35, 40, 50, 71) verschließbar ist,
und wobei der Fluidzugang zu der Vorrichtung durch die Öffnung oder durch einen Fluidverbindungsdurchlass vorgesehen ist.

2. Topische Stickoxid-Liefervorrichtung nach Anspruch 1, wobei die Trägerschicht (100) dafür konstruiert ist, die Erhöhung des Partialdrucks oder der Konzentration von Stickoxid in dem Fluid in der topischen Stickoxid-Liefervorrichtung bereitzustellen.

3. Topische Stickoxid-Liefervorrichtung nach Anspruch 1 oder 2, wobei die Absorptionsschicht (200) ein gelbasiertes Absorptionssystem eines Geliermaterials umfasst.

4. Topische Stickoxid-Liefervorrichtung nach einem vorhergehenden Anspruch, wobei die Absorptionsschicht (200) vollständig benetzt ist.

5. Topische Stickoxid-Liefervorrichtung nach einem vorhergehenden Anspruch, wobei die für Stickoxid durchlässige Schicht (300) nur für Stickoxid durchlässig ist.

6. Topische Stickoxid-Liefervorrichtung nach einem vorhergehenden Anspruch, wobei die Vorrichtung dafür konfiguriert ist, einen Stickoxid-Dosierungspegel zwischen 0,01-100 µmol/(cm²· min) wie etwa 0,1 µmol/(cm² · min) oder zwischen 0,001-10 µmol/(cm²· min) wie etwa 0,05 µmol/(cm² · min) bereitzustellen.

7. Topische Stickoxid-Liefervorrichtung nach einem vorhergehenden Anspruch, wobei die Vorrichtung eine Menge einer Stickoxid-Donorverbindung in dem Bereich von 0,01-10 mg/cm² wie etwa 0,1 mg/cm² oder 0,2 mg/cm² umfasst.

8. Topische Stickoxid-Liefervorrichtung nach einem vorhergehenden Anspruch, wobei die Vorrichtung eine Packung umfasst, die feuchtigkeitsfrei, sauerstofffrei und/oder lichtundurchlässig ist.

9. Topische Stickoxid-Liefervorrichtung nach einem vorhergehenden Anspruch, wobei die für Stickoxid durchlässige Schicht (300) aus einem Material mit mittlerer oder niedriger MVTR, Silikon, Copolyester, EVA, PTFE oder PUR, das eine ausreichende Durchlässigkeit für Stickoxid aufweist, hergestellt ist.

10. Topische Stickoxid-Liefervorrichtung nach einem der Ansprüche 4-9, wobei die Absorptionsschicht (200) einen Fluidgehalt von mehr als 60 % wie etwa 80 % oder mehr als 90 % umfasst.

11. Topische Stickoxid-Liefervorrichtung nach einem vorhergehenden Anspruch, wobei die für Stickoxid durchlässige Schicht (300) eine Hautkontakt-Verbesserungsschicht umfasst oder angrenzend an eine Hautkontakt-Verbesserungsschicht angeordnet ist oder inhärente Hautkontakt-Verbesserungseigenschaften aufweist, wobei sie für Stickoxid durchlässig ist, so dass sie bei Verbindung einen ausreichenden Hautkontakt bereitstellt.

12. Topische Stickoxid-Liefervorrichtung nach Anspruch 11, wobei die Hautkontakt-Verbesserungsschicht eine Klebeschicht wie etwa eine Silikongelschicht, ein Haftklebstoff (PSA) oder eine Hydrogelschicht ist, wobei diese Verbesserungsschicht einen guten Kontakt zwischen der für Stickoxid durchlässigen Schicht (300) und dem Hautbereich bereitstellt.

13. Topische Stickoxid-Liefervorrichtung nach Anspruch 1, die ein Fluidlenksystem umfasst, das dafür ausgelegt ist, das Aktivierungsfluid zu empfangen und das Aktivierungsfluid zu der NO-Donorverbindung zu lenken, wobei das Fluidlenksystem die Form eines Verteilungssystems aufweist und wenigstens einen Fluidverbindungsdurchlass zum Empfangen des Aktivierungsfluids umfasst.

14. Topische Stickoxid-Liefervorrichtung nach Anspruch 1, wobei das Aktivierungsfluid vor der Verwendung getrennt von der Vorrichtung gelagert ist.

15. Topische Stickoxid-Liefervorrichtung nach Anspruch 1, wobei mindestens ein Teil der Absorptionsschicht durch die Öffnung austauschbar ist.

16. Verwendung eines Stickoxids bei der Herstellung eines Medikaments, das
eine für Stickoxid im Wesentlichen undurchlässige Trägerschicht (100) umfasst, die dafür ausgelegt und konfiguriert ist, einen Verlust der Stickoxidkonzentration während der Freisetzung des Stickoxids in dem Medikament zu begrenzen;
eine für Stickoxid durchlässige Schicht (300), die in Verwendung des Medikaments in Richtung eines zu behandelnden Bereichs orientiert ist, wobei die für Stickoxid durchlässige Schicht (300) eine für Stickoxid durchlässige Membran ist;
ein Stickoxid-Erzeugungssystem, das ein Stickoxid freisetzendes Fluid umfasst, wobei das Stickoxid freisetzende Fluid eine Stickoxid-Donorverbindung und ein Aktivierungsfluid umfasst; und
eine Absorptionsschicht (200) zwischen der Trägerschicht (100) und der für Stickoxid durchlässigen Schicht (300), die dafür ausgelegt ist, das Stickoxid freisetzende Fluid darin zu halten,
derart, dass aus dem Stickoxid freisetzenden Fluid freigesetztes Stickoxid innerhalb der Liefervorrichtung und durch die für Stickoxid wahlweise durchlässige Schicht (300) durch eine Zunahme des Partialdrucks oder der Konzentration des Stickoxids darin in Richtung eines Hautbereichs gelenkt wird;
wobei die Trägerschicht (100) eine abdichtend wiederverschließbare oder eine verschließbare Öffnung umfasst, wobei die Öffnung ein Aktivierungsfenster umfasst, das durch einen Trägerschichtdeckel (35, 40, 50, 71) verschließbar ist,
und wobei der Fluidzugang zu der Vorrichtung durch die Öffnung oder durch einen Fluidverbindungsdurchlass vorgesehen ist.

17. Verfahren zur kosmetischen Behandlung, wobei das Verfahren das Liefern eines Stickoxids umfasst, wobei das Verfahren umfasst:
Aktivieren der Stickoxidfreisetzung aus einem Stickoxid-Donorsystem in einer Stickoxid-Liefervorrichtung nach einem der Ansprüche 1-15;
Anordnen der aktivierten Stickoxid-Liefervorrichtung in Kontakt mit einem Hautbereich eines Körpers, wobei eine für Stickoxid durchlässige Schicht (300) der Liefervorrichtung in Richtung des Hautbereichs orientiert ist; und topisches Liefern von Stickoxid von der Stickoxid-Liefervorrichtung an den Hautbereich über die für Stickoxid durchlässige Schicht (300), wobei eine Durchlässigkeit der für Stickoxid durchlässigen Schicht (300) in Abhängigkeit von einem Toxizitätsgrad des Stickoxid-Donorsystems wahlweise gewählt wird, wobei der Hautbereich eine Kosmetikbehandlungsstelle wie etwa eine Stelle von Narben, die durch das Stickoxid verringert werden sollen, oder eine Stelle schlaffer Haut oder von Falten, die durch das Stickoxid gestrafft werden sollen, ist.

18. Verfahren nach Anspruch 17, das das Freisetzen des Stickoxids aus dem Stickoxid-Donorsystem in der Weise, dass ein Partialdruck und somit eine Konzentration des gelösten Stickoxids in der Stickoxid-Liefervorrichtung für die Lieferung des Stickoxids zunehmen, umfasst.

19. Verfahren nach Anspruch 18, das das Befestigen der Stickoxid-Liefervorrichtung an dem Hautbereich mittels einer Hautkontakt-Verbesserungsschicht, die für Stickoxid durchlässig ist, umfasst.

20. Verfahren nach Anspruch 18, wobei das Stickoxid-Donorsystem eine absorbierende Einheit umfasst, die mit einer Stickoxid-Donorverbindung des Stickoxid-Donorsystems zusammenwirkt, wobei sie eine minimale Wanderung der Stickoxid-Donorverbindung aus dem Fleck und das Einfangen von Resten der Stickoxid-Donorverbindung nach dem Niederschlag darin sicherstellt.

## Revendications

1. Dispositif de délivrance d'oxyde nitrique topique comprenant
une couche de support (100) essentiellement imperméable à l'oxyde nitrique, agencée et configurée pour limiter une perte de concentration en oxyde nitrique au cours de la libération d'oxyde nitrique dans le dispositif de délivrance ;
une couche perméable à l'oxyde nitrique (300) en cours d'utilisation du dispositif de délivrance orientée vers une zone devant être traitée, dans lequel ladite couche perméable à l'oxyde nitrique (300) est une membrane perméable à l'oxyde nitrique ;
un système de génération d'oxyde nitrique comprenant un fluide de libération d'oxyde nitrique, dans lequel ledit fluide de libération d'oxyde nitrique comprend un composé donneur d'oxyde nitrique et un fluide d'activation, dans lequel ledit fluide d'activation est destiné à l'activation d'une libération d'oxyde nitrique dudit composé donneur d'oxyde nitrique compris dans ledit dispositif de délivrance d'oxyde nitrique topique ; et
une couche d'absorption (200) entre ladite couche de support (100) et ladite couche perméable à l'oxyde nitrique (300), agencée pour retenir ledit fluide de libération d'oxyde nitrique en son sein,
de telle sorte que l'oxyde nitrique libéré dudit fluide de libération d'oxyde nitrique est dirigé au sein du dispositif de délivrance et à travers la couche perméable à l'oxyde nitrique (300) vers une zone cutanée par une augmentation de la pression partielle ou concentration en oxyde nitrique en son sein ;
dans lequel ladite couche de support (100) comprend une ouverture refermable de manière étanchéifiante, ou fermable, ladite ouverture comprend une fenêtre d'activation qui est fermable par un couvercle de support (35, 40, 50, 71),
et dans lequel un accès au fluide dans le dispositif est fourni par ladite ouverture ou un orifice de communication fluidique.

2. Dispositif de délivrance d'oxyde nitrique topique selon la revendication 1, dans lequel ladite couche de support (100) est conçue pour fournir ladite augmentation de pression partielle ou concentration en oxyde nitrique dans ledit fluide dans ledit dispositif de délivrance d'oxyde nitrique topique.

3. Dispositif de délivrance d'oxyde nitrique topique selon la revendication 1 ou 2, dans lequel ladite couche d'absorption (200) comprend un système d'absorption à base de gel d'un matériau gélifiant.

4. Dispositif de délivrance d'oxyde nitrique topique selon l'une quelconque des revendications précédentes, dans lequel ladite couche d'absorption (200) est entièrement mouillée.

5. Dispositif de délivrance d'oxyde nitrique topique selon l'une quelconque des revendications précédentes, dans lequel ladite couche perméable à l'oxyde nitrique (300) est uniquement perméable à l'oxyde nitrique.

6. Dispositif de délivrance d'oxyde nitrique topique selon l'une quelconque des revendications précédentes, dans lequel le dispositif est configuré pour fournir un niveau de dosage d'oxyde nitrique compris entre 0,01 et 100 µmol/(cm2*min), tel que 0,01 µmol/(cm2*min), ou entre 0,001 et 10 µmol/(cm2*min), tel que 0,05 µmol/(cm²*min).

7. Dispositif de délivrance d'oxyde nitrique topique selon l'une quelconque des revendications précédentes, dans lequel le dispositif comprend une quantité d'un composé donneur d'oxyde nitrique dans la plage de 0,01 à 10 mg/cm2, telle que 0,1 mg/cm2 ou 0,2 mg/cm2.

8. Dispositif de délivrance d'oxyde nitrique topique selon l'une quelconque des revendications précédentes, dans lequel le dispositif comprend un emballage qui est exempt d'humidité, exempt d'oxygène et/ou intransmissible à la lumière.

9. Dispositif de délivrance d'oxyde nitrique topique selon l'une quelconque des revendications précédentes, dans lequel ladite couche perméable à l'oxyde nitrique (300) est constituée d'un matériau à MVTR moyen ou faible, de silicium, de co-polyester, d'EVA, de PTFE ou de PUR qui a une perméabilité suffisante à l'oxyde nitrique.

10. Dispositif de délivrance d'oxyde nitrique topique selon l'une quelconque des revendications 4 à 9, dans lequel ladite couche d'absorption (200) comprend une teneur en fluide de plus de 60 %, telle que 80 % ou plus de 90 %.

11. Dispositif de délivrance d'oxyde nitrique topique selon l'une quelconque des revendications précédentes, dans lequel ladite couche perméable à l'oxyde nitrique (300) comprend une couche d'amplification de contact cutané, ou est agencée de manière adjacente à une couche d'amplification de contact cutané ou a des propriétés d'amplification de contact cutané inhérentes, qui est perméable à l'oxyde nitrique, de sorte qu'elle fournit un contact cutané suffisant dès l'utilisation.

12. Dispositif de délivrance d'oxyde nitrique topique selon la revendication 11, dans lequel ladite couche d'amplification de contact cutané est une couche adhésive, telle qu'une couche en gel de silicium, un adhésif sensible à la pression (PSA) ou une couche d'hydrogel, cette couche d'amplification fournit un bon contact entre ladite couche perméable à l'oxyde nitrique (300) et ladite zone cutanée dès l'utilisation.

13. Dispositif de délivrance d'oxyde nitrique topique selon la revendication 1, comprenant un système de direction de fluide qui est agencé pour recevoir le fluide d'activation et diriger le fluide d'activation vers le composé donneur de NO, le système de direction de fluide est sous la forme d'un système de dispersion et comprend au moins un orifice de connexion fluidique pour recevoir ledit fluide d'activation.

14. Dispositif de délivrance d'oxyde nitrique topique selon la revendication 1, dans lequel le fluide d'activation est stocké séparément du dispositif avant utilisation.

15. Dispositif de délivrance d'oxyde nitrique topique selon la revendication 1, dans lequel au moins une partie de ladite couche absorbante est échangeable à travers ladite ouverture.

16. Utilisation d'oxyde nitrique dans la fabrication d'un médicament comprenant
une couche de support (100) essentiellement imperméable à l'oxyde nitrique, agencée et configurée pour limiter une perte de concentration en oxyde nitrique au cours de la libération d'oxyde nitrique dans le médicament ;
une couche perméable à l'oxyde nitrique (300) en cours d'utilisation du médicament orientée vers une zone devant être traitée, dans laquelle ladite couche perméable à l'oxyde nitrique (300) est une membrane perméable à l'oxyde nitrique ;
un système de génération d'oxyde nitrique comprenant un fluide de libération d'oxyde nitrique, dans laquelle ledit fluide de libération d'oxyde nitrique comprend un composé donneur d'oxyde nitrique et un fluide d'activation ; et
une couche d'absorption (200) entre ladite couche de support (100) et ladite couche perméable à l'oxyde nitrique (300), agencée pour retenir ledit fluide de libération d'oxyde nitrique en son sein,
de telle sorte que l'oxyde nitrique libéré dudit fluide de libération d'oxyde nitrique est dirigé au sein du dispositif de délivrance et à travers la couche sélectivement perméable à l'oxyde nitrique (300) vers une zone cutanée par une augmentation de la pression partielle ou concentration en oxyde nitrique en son sein ;
dans laquelle ladite couche de support (100) comprend une ouverture refermable de manière étanchéifiante, ou fermable, ladite ouverture comprend une fenêtre d'activation qui est fermable par un couvercle de support (35, 40, 50, 71),
et dans laquelle un accès au fluide dans le dispositif est fourni par ladite ouverture ou un orifice de communication fluidique.

17. Procédé de traitement cosmétique comprenant la délivrance d'oxyde nitrique, ledit procédé comprenant :
activer la libération d'oxyde nitrique d'un système donneur d'oxyde nitrique dans un dispositif de délivrance d'oxyde nitrique selon l'une quelconque des revendications 1 à 15 ;
placer le dispositif de délivrance d'oxyde nitrique activé en contact avec une zone cutanée d'un corps, avec une couche perméable à l'oxyde nitrique (300) du dispositif de délivrance orientée vers ladite zone cutanée ; et délivrer l'oxyde nitrique de manière topique dudit dispositif de délivrance d'oxyde nitrique à ladite zone cutanée par le biais de ladite couche perméable à l'oxyde nitrique (300), dans lequel
une perméabilité de ladite couche perméable à l'oxyde nitrique (300) est choisie sélectivement en fonction d'un niveau de toxicité dudit système donneur d'oxyde nitrique, dans lequel ladite zone cutanée est un site de traitement cosmétique, tel qu'un site de cicatrices devant être réduites par ledit oxyde nitrique, ou un site de peau affaissée ou de rides devant être lissées par ledit oxyde nitrique.

18. Procédé selon la revendication 17, comprenant la libération dudit oxyde nitrique dudit système donneur d'oxyde nitrique de sorte qu'une pression partielle et ainsi une concentration en oxyde nitrique dissous dans ledit dispositif de délivrance d'oxyde nitrique augmentent pour ladite délivrance d'oxyde nitrique.

19. Procédé selon la revendication 18, comprenant fixer ledit dispositif de délivrance d'oxyde nitrique à ladite zone cutanée au moyen d'une couche d'amplification de contact cutané perméable à l'oxyde nitrique.

20. Procédé selon la revendication 18, dans lequel ledit système donneur d'oxyde nitrique comprend une unité absorbante interagissant avec un composé donneur d'oxyde nitrique dudit système donneur d'oxyde nitrique, assurant une migration minimale du composé donneur d'oxyde nitrique hors du timbre et capturant des résidus dudit composé donneur d'oxyde nitrique après précipitation en son sein.
